# EUROPÄISCHE PATENTANMELDUNG

(11) **EP 4 120 116 A1**
(43) Veröffentlichungstag der Anmeldung: **18.01.2023**
(21) Anmeldenummer: 21184956.7
(22) Anmeldetag: 12.07.2021
(51) Int. Cl.: G06F 30/27, A61C 13/00, A61C 1/08, A61C 7/00, G06F 113/10

(54) **BESTIMMUNG MIT NEURONALEN NETZEN VON ATTRIBUTEN VON DENTALEN BAUTEILEN IN ADDITIVER/SUBTRAKTIVER HERSTELLUNG**

(71) Anmelder: DENTSPLY SIRONA Inc., York, PA 17401 (US); Sirona Dental Systems GmbH, 64625 Bensheim (DE)
(72) Erfinder: Stahl, Christian, 64625 Bensheim (DE)
(74) Vertreter: Özer, Alpdeniz

(57) **Zusammenfassung**

Die vorliegende Erfindung betrifft ein Verfahren zur hochwertigen automatischen Vorbereitung von additiven/subtraktiven Herstellungsaufträgen für dentale Bauteile wie z.B. Schienen, Prothesenbasen, Modellen, Restaurationen wie Brücken und Kronen, gekennzeichnet dadurch, dass dieses neuronale Netze für die Bauteiltypklassifizierung verwendet, wobei für jeden Bauteiltyp ein spezialisiertes vortrainiertes neuronales Netz für Oberflächen- und/oder Volumenattributierung des Bauteils verwendet wird, wobei die Oberflächen- und Volumenattribute die Genauigkeits- und Qualitätsanforderungen der Konstruktionselemente der Bauteile hinsichtlich der vorgesehenen Nutzung beschreiben, wobei auf Basis der Bauteiltypklassifizierung dann Oberflächen- und/oder Volumenattribute gesetzt werden.

## Beschreibung

### TECHNISCHES GEBIET DER ERFINDUNG

Die vorliegende Erfindung bezieht sich auf additive/subtraktive Herstellungsverfahren von dentalen Bauteilen.

### HINTERGRUND DER ERFINDUNG

Die aus dem Stand der Technik bekannte CAD/CAM Software für dentale Bauteile verwenden Oberflächenattribute, um eine hochwertige Vorbereitung von 3D-Druckaufträgen anzubieten. Der Benutzer kann mit der CAD/CAM Software diese Oberflächenattribute manuell definieren. Diese Oberflächenattribute markieren z.B. sensible/funktionale Oberflächenregionen, auf denen keine Supports beim 3D Drucken angebracht werden sollen, um eine manuelle Nachbearbeitung auf diesen Regionen zu vermeiden. Wenn diese Oberflächenattribute nicht bereits mit der CAD/CAM-Software gesetzt wurden, was häufig der Fall ist, müssen Sie nachträglich vervollständigt werden. Für Fremdimporte, insbesondere im stl-Format, muss der Benutzer diese Oberflächenattribute manuell hinzufügen. Dafür steht z.B. in einiger CAD/CAM Software ein "Painter"-Tool bereit. Der Vorgang des manuellen Attributierens mittels "Painter-Tool" ist jedoch zeitaufwändig und potentiell fehleranfällig, da der Benutzer die Oberflächenattribute nach eigenem Ermessen und nicht zwangsläufig gemäß den vorgesehenen Aspekten definiert.

### OFFENBARUNG DER ERFINDUNG

Ein Ziel der vorliegenden Erfindung ist die Bereitstellung eines Verfahrens sowie einer CAD/CAM Software zur hochwertigen automatischen Vorbereitung von additiven/subtraktiven Herstellungsaufträgen für dentale Bauteile.

Ein weiteres Ziel der vorliegenden Erfindung ist die Bereitstellung eines Verfahrens sowie einer CAD/CAM Software, mit denen die Oberflächen- und Volumenattribute eines Bauteils durch neuronale Netze berechnet werden.

Diese Ziele werden durch das Verfahren nach Anspruch 1 und die CAD/CAM Software nach Anspruch 7 erreicht. Die Gegenstände der abhängigen Ansprüche beziehen sich auf Weiterentwicklungen sowie bevorzugte Ausführungsformen.

Das erfindungsgemäße Verfahren und die entsprechende CAD/CAM Software dienen zur hochwertigen automatischen Vorbereitung von additiven/subtraktiven Herstellungsaufträgen für dentale Bauteile z.B. Schienen, Prothesenbasen, Modellen, Restaurationen wie u.a. Brücken und Kronen. Die CAD/CAM Software weist neuronale Netze für die Bauteiltypklassifizierung auf. Für jeden Bauteiltyp wird ein spezialisiertes vortrainiertes neuronales Netz für die Oberflächen- und/oder Volumenattributierung des Bauteils verwendet. Die Oberflächen- und Volumenattribute beschreiben sensible/funktionale Oberflächen und Volumina, insbesondere die Genauigkeits- und Qualitätsanforderungen der Konstruktionselemente der Bauteile. Auf Basis der Bauteiltypklassifizierung werden die Oberflächen- und/oder Volumenattribute gesetzt.

Als Trainingsdaten können Test- und Kundenfälle aus einer CAD/CAM-Software dienen, in denen die Oberflächen- und Volumenattribute mit der CAD/CAM-Software auf Basis von Konstruktionselementen fachgerecht gesetzt werden.

Eine vorteilhafte Wirkung der Erfindung ist, dass durch das Verfahren bzw. die CAD/CAM Software der Vorgang des Attributierens von Oberflächen- und Volumenattributen automatisch mittels neuronale Netze gemäß der vorgesehenen Aspekte des Bauteils fachgerecht erfolgen kann. Die CAD/CAM Software spart Zeit und ist weniger fehleranfällig wegen der Nutzung von vortrainierten neuronalen Netzen.

### KURZBESCHREIBUNG DER ZEICHNUNG

In der nachfolgenden Beschreibung wird die vorliegende Erfindung anhand von Ausführungsbeispielen unter Bezugnahme auf die Zeichnung näher erläutert, wobei Abb.l - zeigt ein Bauteil mit Konstruktionselementen nach einer Ausführungsform der Erfindung.

Die in der Zeichnung gezeigten Referenznummern bezeichnen die unten aufgeführten Elemente, auf die in der nachfolgenden Beschreibung der beispielhaften Ausführungsformen Bezug genommen wird.
- 1.: Bauteil
- 2,2',2": Konstruktionselement
- 3.: Führungshülse

Im Folgenden wird das erfindungsgemäße Verfahren näher erläutert. Das erfindungsgemäße Verfahren wird als CAD/CAM Software implementiert.

Durch die erfindungsgemäße CAD/CAM Software können 3D Druck- oder Fräsaufträge für dentale Bauteile z.B. wie Schienen, Prothesenbasen, Modelle, Restaurationen, Brücken und Kronen, hochwertig automatisch vorbereitet werden. Die CAD/CAM Software verwendet neuronale Netze für die Bauteiltypklassifizierung u.a. Restaurationstypklassifizierung. Für jeden Bauteiltyp wird ein spezialisiertes vortrainiertes neuronales Netz für die Oberflächen- und/oder Volumenattributierung des Bauteils verwendet. Die Oberflächen- und Volumenattribute beschreiben die gewünschte bzw. notwendige Genauigkeitsanforderungen und Qualitätsanforderungen der Konstruktionselemente der Bauteile. Die Oberflächen- und/oder Volumenattribute werden auf Basis der Bauteiltypklassifizierung gesetzt. Genauigkeitsanforderungen und Qualitätsanforderungen umfassen Eigenschaften wie Maßhaltigkeit, mechanische Festigkeit, Oberflächenpräzision, und die Vermeidung von Anbringung von Stützelementen beim 3D Druck usw.

Die Konstruktionselemente können auch innerhalb der Variationen eines Bauteiltyps charakteristische Eigenschaften haben wie Morphologie, Position innerhalb des Bauteils, Umgebungsmorphologie, anhand derer sie sich mithilfe der neuronalen Netze klassifizieren lassen.

Die Konstruktionselemente sind z.B. Bohrlöffelauflage bei einer Bohrschablone, Sockel bei Modellen, Zahntaschen bei Prothesenbasen usw.

Als Trainingsdaten können Test- und Kundenfälle aus der CAD/CAM-Software dienen, in denen die Oberflächen- und Volumenattribute von der CAD/CAM-Software auf Basis von Konstruktionselementen fachgerecht gesetzt werden.

Die Bauteiltypklassifizierung kann vorzugsweise auf Basis von Triangulationsknoten und/oder Dreiecken der Bauteile erfolgen.

Die Datensätze, die aus dem Verfahren bzw. aus der CAD/CAM Software resultieren, werden zum Trainieren eines oder mehrerer neuronaler Netze verwendet. Die Datensätze können auch zur Visualisierung auf einer Anzeige gezeigt werden.

Die CAD/CAM Software wird auf einem Speichermedium als Programmcode bereitgestellt und kann auf einem Rechnersystem durchgeführt werden. Die CAD/CAM Software kann vorzugsweise auch das additive/subtraktive Herstellungsgerät z.B., einen 3D Drucker oder eine Fräsmaschine steuern. Das Rechnersystem umfasst vorzugsweise eine Benutzeroberfläche für die Eingabe von Daten, die die Bauteile, die Geometrie und die Konstruktionselemente beschreiben und/oder von Trainingsdaten, die für die Bauteile relevant sind.

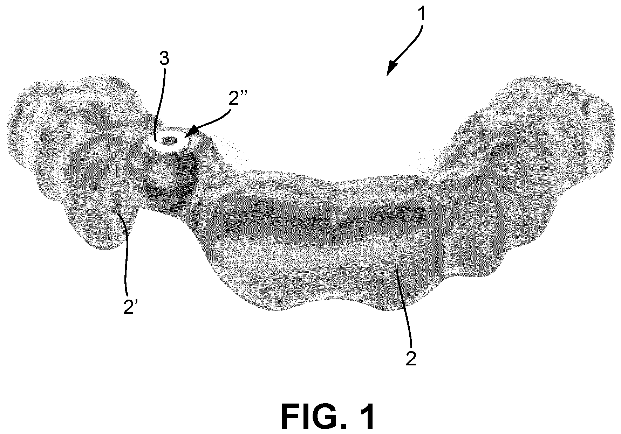

Abb1. zeigt ein Bauteil (1), insbesondere eine Bohrschablone. Die Bohrschablone hat eine Öffnung für die Aufnahme einer Führungshülse (3) wo ein Bohrer oder ein endodontischer Pfeiler geführt werden kann. An den Stellen, an denen die Führungshülse (3) aufliegt, kann das Oberflächenattribut z.B. die Genauigkeitsanforderung für die Herstellung als hoch festgelegt werden um eine präzise Platzierung der Führungshülse (3) zu ermöglichen. Die unterschiedlichen Konstruktionselemente (2,2',2") der Bohrschablone umfassen jeweils z.B. die Unterfläche der Bohrschablone, die Oberfläche der Bohrschablone und die Innenfläche der Öffnung in der Bohrschablone welche als Auflagefläche für die Führungshülse (3) dient. An der Oberfläche der Bohrschablone, im Gegensatz zu der Unterfläche, wo die Bohrschablone auf dem Zahn aufliegt, kann das Oberflächenattribut z.B. die Genauigkeitsanforderung relativ niedrig festgelegt werden. Die vortrainierte neuronale Netze erkennen automatisch das Bauteil (1), die Konstruktionselemente (2,2',2") und die entsprechend Genauigkeitsanforderung anhand der Vorgesehenen Nutzungen, für das entsprechende Herstellverfahren.

Anstelle von Oberflächenattributen oder zusätzlich zu Oberflächenattributen können auf ähnliche Weise auch Volumenattribute verwendet werden wobei die additive/subtraktive Herstellung z.B. Fräsen oder 3D Drucken der Volumenbereiche mit den entsprechenden Volumenattribute, die die Genauigkeitsanforderung beschreiben durchgeführt werden.

In einer weiteren Ausführungsform ermöglicht es die CAD/CAM-Software bei nicht ausgehöhlten Modellen den auszuhöhlenden Bereich zu markieren. Anschließend setzen die vortrainierten neuronalen Netze die Oberflächen- und/oder Volumenattribute des auszuhöhlenden Bereichs auf Basis der Bauteiltypklassifizierung.

Oberflächen- und/oder Volumenattribute können Werte bzw. Merkmale annehmen, die die Genauigkeits- und Qualitätsanforderung für die additive/subtraktive Herstellung bestimmen z.B. Schichtstärke, Glätte, Rauheit, Belichtungsdosis, Maskentyp, Werkzeugtyp, Anwesenheit/Abwesenheit von Stützstrukturen usw.

## Patentansprüche

1. Verfahren zur hochwertigen automatischen Vorbereitung von additiven/subtraktiven Herstellungsaufträgen für dentale Bauteile wie z.B. Schienen, Prothesenbasen, Modellen, Restaurationen wie Brücken und Kronen, **gekennzeichnet dadurch, dass** dieses neuronale Netze für die Bauteiltypklassifizierung verwendet,
wobei für jeden Bauteiltyp ein spezialisiertes vortrainiertes neuronales Netz für Oberflächen- und/oder Volumenattributierung des Bauteils verwendet wird,
wobei die Oberflächen- und Volumenattribute die Genauigkeits- und Qualitätsanforderungen der Konstruktionselemente der Bauteile hinsichtlich der vorgesehenen Nutzung beschreiben,
wobei auf Basis der Bauteiltypklassifizierung dann Oberflächen- und/oder Volumenattribute gesetzt werden.

2. Verfahren nach Anspruch 1, **gekennzeichnet dadurch, dass** die Konstruktionselemente auch innerhalb der Variationen eines Bauteiltyps charakteristische Eigenschaften haben wie Morphologie, Position innerhalb des Bauteils, Umgebungsmorphologie, anhand derer sie sich mithilfe des neuronalen Netzwerks klassifizieren lassen.

3. Verfahren nach Anspruch 1 oder 2, **gekennzeichnet dadurch, dass** das Konstruktionselement mindestens eines der folgenden ist:
Bohrlöffelauflage bei der Bohrschablone,
Sockel bei Modellen,
Zahntasche bei Prothesenbasen.

4. Verfahren nach einem der vorangegangenen Ansprüche, **gekennzeichnet dadurch, dass** als Trainingsdaten Test- und Kundenfälle aus einer CAD/CAM-Software dienen, in denen die Oberflächen- und Volumenattribute mit der CAD/CAM-Software auf Basis von Konstruktionselementen gesetzt werden.

5. Verfahren nach einem der vorangegangen Ansprüche, **gekennzeichnet dadurch, dass** die Bauteiltypklassifizierung auf Basis von Triangulationsknoten und/oder Dreiecken der Bauteile erfolgt.

6. Verwendung von Datensätzen, die aus einem der vorangegangenen Ansprüche resultieren, zum Trainieren eines oder mehrerer neuronaler Netze und/oder zur Visualisierung auf einer Anzeige.

7. CAD/CAM Software, die einen computerlesbaren Programmcode umfasst, der wenn er auf einem Rechner oder neuronalen Netz ausgeführt wird, den Rechner veranlasst die Verfahrensschritte nach einem der vorangegangenen Ansprüche auszuführen.

8. Speichermedium, das die CAD/CAM Software nach Anspruch 7 umfasst.
